# EUROPEAN PATENT APPLICATION

(11) **EP 1 775 340 A1**
(43) Date of publication of application: **18.04.2007**
(21) Application number: 05734077.0
(22) Date of filing: 15.04.2005
(51) Int. Cl.: C12N 5/06

(54) **METHOD OF CELL TRANSDIFFERENTIATION**

(30) Priority: 16.04.2004 JP 2004121350
(71) Applicant: HITACHI MEDICAL CORPORATION, Tokyo 101-0047 (JP); Satomura, Kazuhito, Tokushima 7708051 (JP)
(72) Inventor: Satomura, Kazuhito, 504, Sunrise Avenue, Tokushima-shi, Tokushima 7708051 (JP)
(74) Representative: Strehl, Peter
(86) International application number: PCT/JP2005/007655
(87) International publication number: WO 2005/100550

(57) **Abstract**

An object of the present invention is to provide a method for transdifferentiating a mature cell without using a DNA methylating agent or a DNA demethylating agent. The present invention provides a method for transdifferentiating a finally differentiated mature cell having a first differentiation phenotype into a finally differentiated mature cell having a second differentiation phenotype that differs from the above first differentiation phenotype by changing culture conditions for the finally differentiated mature cell having the first differentiation phenotype, wherein culture conditions suitable for culturing the finally differentiated mature cell having the above first differentiation phenotype are changed to culture conditions suitable for culturing the finally differentiated mature cell having the above second differentiation phenotype.

## Description

### Technical Field

The present invention relates to a method for transdifferentiating cells. More specifically, the present invention relates to a method for transdifferentiating cells by varying the environment for culturing finally differentiated mature cells. The present invention further relates to differentiated cells that can be obtained by the above method.

### Background Art

Securing of a source for supplying target cells is essential in any field, based on which regenerative medicine or cellular medicine is realized. Various techniques therefor, such as techniques using stem cells or mature cells, have been developed to date.

Currently, a merit of pluripotent stem cells represented by embryonic stem cells (ES cells) and mesenchymal stem cells (MSCs) that are mainly used in the field is their pluripotency. At the same time, such cells have many problems, such that control of such cells is difficult because of their pluripotency.

In the meantime, transdifferentiation technology for cells other than pluripotent hepatocytes, that is, for cells, the differentiation direction of which is determined to some extent (e.g., tissue stem cells and undifferentiated tissue cells), has been reported in recent years.

A typical transdifferentiation technology that has been reported to date is a method that uses a DNA-methylating agent or a DNA demethylating agent. This technology uses a property of DNA whereby DNA transcription is regulated by a change in the ability of an intranuclear transcription factor to bind to the DNA due to methylation or demethylation. Specifically, this technology forces cell transdifferentiation to take place by causing a methylating agent or a demethylating agent to directly act on DNA in the cells, so as to artificially alter the ability of a transcription factor to bind to the DNA.

Fukuda et al. applied this technology to mesenchymal stem cells. Specifically, they added 5-azacytidine (a demethylating agent) to mesenchymal cells and then succeeded in obtaining cardiac muscle cells from a differentiation induction system of mesenchymal stem cells (Experimental Medicine, Vol. 19, No. 12 (August issue), 2001). Furthermore, it has been reported that mesenchymal stem cells are differentiated by this technique into various cells including osteoblasts, chondrocytes, and fat cells, in addition to cardiac muscle cells.

With this technology, currently, cell transdifferentiation can be caused to take place successfully by causing DNA methylation or DNA demethylation, but differentiation direction cannot be controlled. When differentiation is caused using these agents, differentiation direction (which the cells (subjected to methylation or demethylation) are differentiated into) is randomly determined. Under current circumstances, necessary cells are isolated from many cells that have randomly changed, following which they are used. Accordingly, the technology not only requires much time and effort to obtain a target cell, but also involves the risk that cells could undergo unpredicted mutation. Thus, this technology is extremely risky in medical applications or industrial uses.

Furthermore, as an example of transdifferentiation, JP Patent Publication (Kokai) No. 2003-304878 discloses a technology that involves transdifferentiating fetal mammalian hepatocytes or hepatic progenitor cells so as to induce insulin-producing cells. With this technology, insulin-producing cells are induced through culture in the presence of nicotinic acid amide or gene introduction without using the above-described transdifferentiation agent. However, this technology is merely a technology that uses undifferentiated cells such as fetal cells or progenitor cells.

### Disclosure of the Invention

An object to be achieved by the present invention is to address the above-described problems of conventional technologies. Specifically, an object to be achieved by the present invention is to provide a method for transdifferentiating a mature cell without using a DNA methylating agent or a DNA demethylating agent.

As a result of intensive studies to achieve the above object, the present inventors have demonstrated that a finally differentiated mature cell having a first property can be transdifferentiated into a finally differentiated mature cell having a second property that differs from the above first property by changing culture conditions for the finally differentiated mature cell having the first property. The present inventors have completed the present invention based on these findings.

Thus, the present invention provides a method for transdifferentiating a finally differentiated mature cell having a first differentiation phenotype into a finally differentiated mature cell having a second differentiation phenotype that differs from the above first differentiation phenotype by changing culture conditions for the finally differentiated mature cell having the first differentiation phenotype, wherein culture conditions suitable for culturing the finally differentiated mature cell having the above first differentiation phenotype are changed to culture conditions suitable for culturing the finally differentiated mature cell having the above second differentiation phenotype.

Preferably, the change of the culture conditions is a change of a medium.

Preferably, transdifferentiation is performed without using a DNA methylating agent or a DNA demethylating agent.

Preferably, the finally differentiated mature cell having the first differentiation phenotype is an osteoblast, and the finally differentiated mature cell having the second differentiation phenotype is a nerve cell. In this case, preferably, transdifferentiation is performed by exchanging a medium containing β-glycerophosphate, ascorbic acid, and Glutamax with a medium containing bFGF, FGF-8, EGF, and BDNF.

According to another aspect of the present invention, a finally differentiated mature cell having a predetermined differentiation phenotype which is obtained by the above method is provided.

### Brief Description of the Drawings

Fig. 1 shows the appearance of cells transdifferentiated from osteoblasts into nerve cells according to the method of the present invention.
Fig. 2 shows the result of immunostaining of nerve cells transdifferentiated by the method of the present invention.
Fig. 3 shows the appearance of cells transdifferentiated again from osteoblast-derived nerve cells into the osteoblasts by the method of the present invention.

### Preferred Embodiments of the Invention

Hereafter, embodiments of the present invention are described in detail.

The present invention relates to a method for transdifferentiating a finally differentiated mature cell having a first differentiation phenotype into a finally differentiated mature cell having a second differentiation phenotype that differs from the above first differentiation phenotype by changing culture conditions for the finally differentiated mature cell having the first differentiation phenotype, wherein culture conditions appropriate for culturing the finally differentiated mature cell having the above first differentiation phenotype are changed to culture conditions suitable for culturing the finally differentiated mature cell having the above second differentiation phenotype.

"Transdifferentiation" in this description means that differentiated mature cells having a differentiation phenotype lose the differentiation phenotype and are then changed into differentiated mature cells having another differentiation phenotype.

A methylating agent or demethylatng agent that is a conventional technology concerning transdifferentiation is a factor that promotes the programming of cell differentiation by directly acting on the transcriptional regulatory system of cells the differentiation direction of which has been determined. The method using methylating agent or demethylation agent has the merit of being capable of inducing various cells by single type of agent. In the meantime, such a differentiation direction cannot be determined with this technique. Thus, obtainment of a target cell requires tasks for selection and isolation of the target cell from randomly differentiated cells.

Meanwhile, the method of the present invention realizes transdifferentiation not by forcibly promoting the reprogramming of cells of overall mammals with the use of a transdifferentiation agent, but by adjusting the cell culture environment to an environment appropriate for a target cell. It is believed that this effect is exerted through the induction of cells' original genes via the peripheral environment for the cells as a result of the transfer of the cells into a medium with an appropriate growth environment. Therefore, unlike transdifferentiation that is caused by forcing cell differentiation to take place using a transdifferentiation agent, the technique uses cells' original ability to cope with a culture environment. With the use of this technique, it is possible to differentiate all mammalian cells into target cells. Furthermore, random differentiation that is caused by the use of a demethylating agent seldom takes place. Thus, it becomes possible to use mature cells as a stable source for supplying such cells in regenerative medicine, cellular medicine, or the like.

Moreover, undifferentiated cells (cells before differentiation) can be induced again by returning from conditions for differentiated cells to conditions employed before differentiation. Cell differentiation can be freely controlled for any purpose.

Furthermore, the present invention enables the precise differentiation of cells into target cells. Thus, no task is required for isolation of cells, which is required upon the use of a transdifferentiation agent of conventional technology. Moreover, transdifferentiation task can be realized with only the convenient task of exchanging media. Accordingly, the method of the present invention can also be easily automated and thus also is an industrially excellent technique.

In the meantime, the above-described method disclosed in JP Patent Publication (Kokai) No. 2003-304878 does not use any transdifferentiation agent, but is a technology limited to undifferentiated hepatocytes. The present invention can be implemented for cells of all types of biological species and cell species, as long as they are mammalian cells. Furthermore, gene introduction is employed in JP Patent Publication (Kokai) No. 2003-304878; however, the present invention requires no gene introduction.

Furthermore, the above-described conventional technologies have been reported as transdifferentiation technologies. However, undifferentiated cells such as stem cells or progenitor cells are used as sources for supplying cells in most of these reports. Also in this regard, these conventional technologies differ from the present invention which is a technology for differentiating finally differentiated mature cells into another cell. According to the present invention, another cell can be induced without using an undifferentiated cell. At clinical sites where undifferentiated cells such as stem cells have been used, the present invention enables the use of a finally differentiated mature cell that can be conveniently recovered instead of a stem cell, so as to expand the options for cell selection at clinical sites. With this regard, the technology of the present invention has a major advantage over conventional technologies.

MC3T3-E1 used in the Examples of this description is a typical osteoblast cell line that is recognized and widely used throughout the world. When the technique of the present invention was applied for this cell, the cells were morphologically changed into nerve-cell-like cells, which expressed neurofilament and GFAP which are nerve cell marker. Thus, it was demonstrated that osteoblasts had been transdifferentiated into nerve cells by the technique of the present invention.

Furthermore, when a medium for nerve cells was changed to a medium for osteoblasts, nerve cells were changed into osteoblasts. Thus, it was shown that such transdifferentiation freely occurs also in a backward direction, depending on medium conditions.

Any cell in an organism originally possesses the entirety of the organism's genetic information. Hence, it is believed that in the Example below, the nerve-cell-related genes of osteoblasts, the expression of which is generally arrested, were expressed due to the effects of a factor contained in a medium for nerve cells, following which the differentiation from osteoblasts to nerve cells proceeded. Moreover, when culture conditions for cells that had differentiated into nerve cells were changed, specifically, from the medium for nerve cells to the medium for osteoblasts, differentiation into osteoblasts occurred. It can be said that this differentiation also takes place due to a similar reason.

The present invention is a technology for realizing transdifferentiation by using genetic information that all cells originally possess and making the cell growth environment appropriate for a target cell as described above.

Specifically, all cells possess the entirety of the organism's genetic information. Thus, the application of the transdifferentiation technology of the present invention is not limited to osteoblasts and nerve cells, but can be applied to all mammalian cells.

Types of cells that are used in the present invention are not particularly limited. Preferably, cells of any mammals including humans and non-human mammals can be used. Examples of non-human mammals include, but are not limited to, mice and other rodents, cattle, horses, pigs, goats, sheep, dogs, and cats. Cells that are used in the present invention are particularly preferably human-derived cells. In the present invention, cells to be transdifferentiated can be obtained from tissues derived from ectoderm, mesoderm, endoderm, neural crest, or ectodermal membrane.

According to the method of the present invention, transdifferentiation is performed by changing the culture conditions suitable for culturing finally differentiated mature cells having the first differentiation phenotype into the culture conditions suitable for culturing finally differentiated mature cells having the second differentiation phenotype. "Culture conditions suitable for culturing cells" means that the composition of a medium is suitable for culturing the cells, for example.

Types of cells that can be used or prepared as starter materials in the present invention and specific examples of the compositions of media to be used for culturing cells are as described below. However, cell types and medium compositions that are used in the present invention are not limited to these examples.
(1) Osteoblasts can be generally obtained by transdifferentiation in a medium containing dexamethasone, ascorbate, Mesenchymal Stem Cell Supplement (MCGS), L-glutamine, β-glycerophosphate, and the like. An example of a composition of a medium for osteoblasts is an αMEM medium supplemented with FBS (final concentration: 10%), 5 mM to 10 mM β-glycerophosphate, 50 µg/ml ascorbic acid, and 1 x Glutamax.
(2) Nerve cells can be generally obtained in transdifferentiation in a medium containing a retinoid compound such as retinoic acid or vitamin A, and if desired, a nerve growth factor, neurotrophin or the like such as a brain-derived neurotrophic factor (BDNF), a ciliary neurotrophic factor (CNTF), a platelet-derived growth factor (PDGF), a nerve growth factor (NGF), neurotrophin (NT)-3, neurotrophin (NT)-4, and sonic hedgehog (sonic hedgehog) (Shh). An example of the composition of a medium for nerve cells is an αMEM medium supplemented with FBS (final concentration: 10%), 100 ng/ml bFGF, 10 ng/ml FGF-8, 10 ng/ml EGF, and 10 ng/ml BDNF.
(3) Chondrocytes can be generally obtained by transdifferentiation in a medium containing dexamethasone, ascorbic acid, sodium pyruvate, proline, L-glutamine, transferrin, insulin, TGF-β, and the like.
(4) Fat cells can be generally obtained by transdifferentiation in a medium containing insulin, L-glutamine, dexamethasone, indomethacin, and the like.
(5) Hepatocytes can be generally obtained by transdifferentiation in a medium containing insulin, dexamethasone, ascorbic acid, FGF-4, FGF, HGF, and the like.

Cell culturing in the present invention can be performed using the above suitable media under general culture conditions for mammalian cells (e.g., temperature ranging from room temperature to 37°C and within a 5% CO₂ incubator). Forms of culturing are not particularly limited. For example, static culturing can be performed.

Whether or not transdifferentiation has been performed by the method of the present invention can be determined using the presence or the absence of the expression of a marker that is specific to a target cell as an indicator. The expression of such marker can be detected by biochemical or immunochemical techniques known by persons skilled in the art. For example, immunochemical techniques such as enzyme-linked immunosorbent assay (ELISA), immunofluorescent assay (IFA), immunoelectrophoresis, immunochromatography assay, and immunohistochemical staining method can be used, but examples are not limited thereto. In these methods, marker-specific polyclonal or monoclonal antibodies or fragments thereof, which selectively bind to antigens of various target cells, are used. Antibodies against various specific markers are marketed and can be properly obtained by persons skilled in the art.

Alternatively, the expression of a marker that is specific to a target cell can also be detected by a molecular biological method such as RT-PCR, transcription mediated amplification (TMA), reverse transcriptase-ligase chain reaction (RT-LCR), or hybridization analysis.

Alternatively, the expression of such marker can also be specifically detected by a tissue staining technique such as alizarin red staining, alcian blue staining, Oil-Red-O staining, Von Kossa staining, or indocyanine green staining using metabolic products of differentiated cells, cellular drug metabolism, or properties such as dyeing property.

Specific examples of a marker that is specific to a nerve cell include neurofilament, GFAP (glial fibrillary acidic protein), nestin, neural specific tubulin, and neural specific enolase.

Examples of a marker that is specific to an osteoblast include collagen type I, osteopontin, osteocalcin, MGP (matrix Gla protein), ALP (alkaline phosphatase), and Cbfa1 (core binding factor alpha 1). Examples of a specific staining method include alizarin red staining and Von Kossa staining.

Examples of a marker that is specific to a chondrocyte include collagen type II, chondroitin sulfate-proteoglycan, COMP (cartilage oligomeric matrix protein), aggrecan, and SOX9 (SRY-related HMG box 9). Furthermore, an example of a specific staining method is alcian blue staining.

Examples of a marker that is specific to a fat cell include PPARγ (Peroxisome Proliferator Activated Receptor-gamma) and the like. Furthermore, an example of a specific staining method is Oil-Red-O staining.

Examples of a marker that is specific to a hepatocyte include albumin and cytochrome P450. Furthermore, an example of a specific staining method is indocyanine green staining.

Moreover, transdifferentiation to a target cell can also be confirmed using morphological standards. For example, nerve cells such as neurons or neuron-like cells can be confirmed by the expression of axonal projections or axonal-projection-like projections each having a size 3 times or more greater than the relevant cellular diameter.

The transdifferentiation method according to the present invention is performed *in vitro.* The thus obtained mature cells or tissues or organs generated with the use of this method are transplanted into a body, so that tissues or organs can be regenerated.

The present invention will be further described in detail by referring to the following example. However, the present invention is not limited by the examples.

### Examples

### (1) Method

Mouse osteoblasts of an established line (MC3T3/E1) were cultured to subconfluency in an αMEM medium supplemented with FBS (final concentration: 10%), 5 mM to 10 mM β-glycerophosphate, 50 µg/ml ascorbic acid, and 1 x Glutamax (hereinafter, the medium for osteoblasts).

Cells cultured in the medium for osteoblasts were washed with a DMEM medium. Subsequently, the culture conditions of the cultured MC3T3/E1 were changed from culture using the medium for osteoblasts to culture using an αMEM medium supplemented with FBS (final concentration: 10%), 100 ng/ml bFGF, 10 ng/ml FGF-8, 10 ng/ml EGF, and 10 ng/ml BDNF (hereinafter, the medium for nerve cells). Changes in the cells caused by the change of the media were examined with time through morphological observation by microscopic examination and through the analysis of protein expression by immunostaining.

### (2) Result

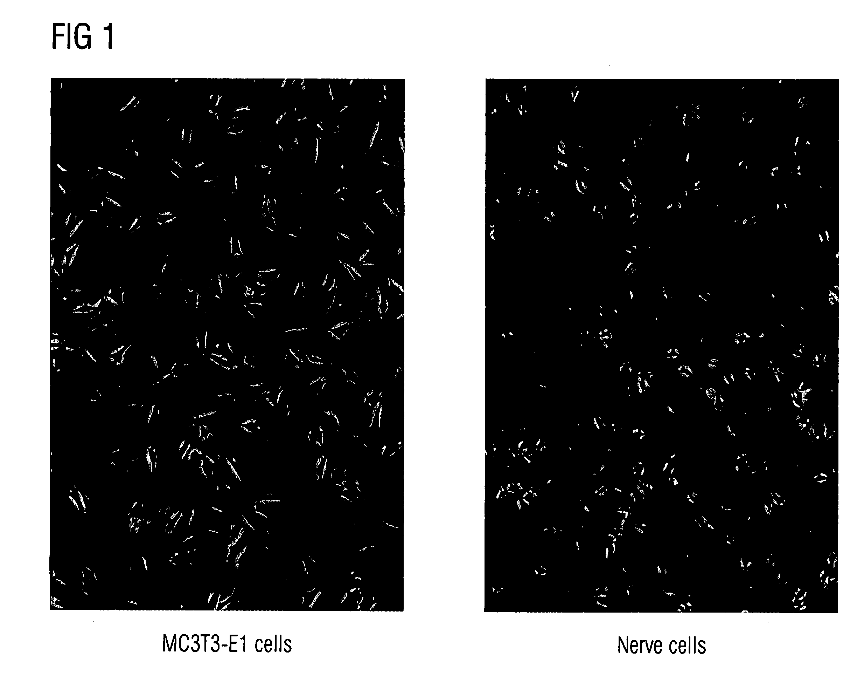

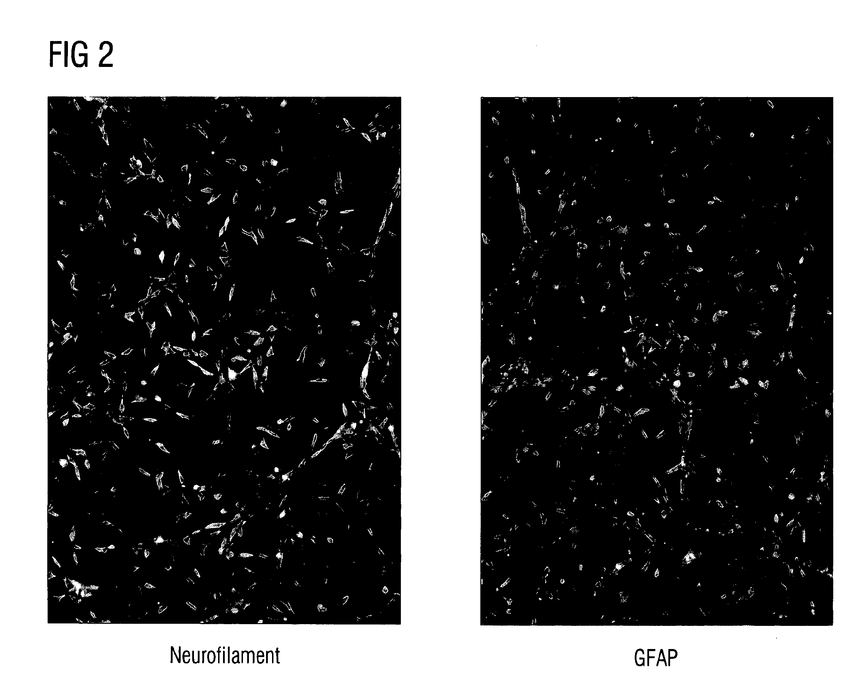

MC3T3/E1 cells cultured in the medium for osteoblasts were transferred to the medium for nerve cells. 6 to 12 hours later, a pavement-stone-like shape unique to osteoblasts was changed to a nerve-cell-like shape with long cellular processes (Fig. 1). The resultant cells with such nerve-cell-like shape were subjected to immunostaining using antibodies against neurofilament and GFAP (glial fibrillary acidic protein), which are marker proteins specific to nerve cells. As a result, nerve-cell-like cells were stained (Fig. 2). From the above result, it was shown that these cells were nerve cells.

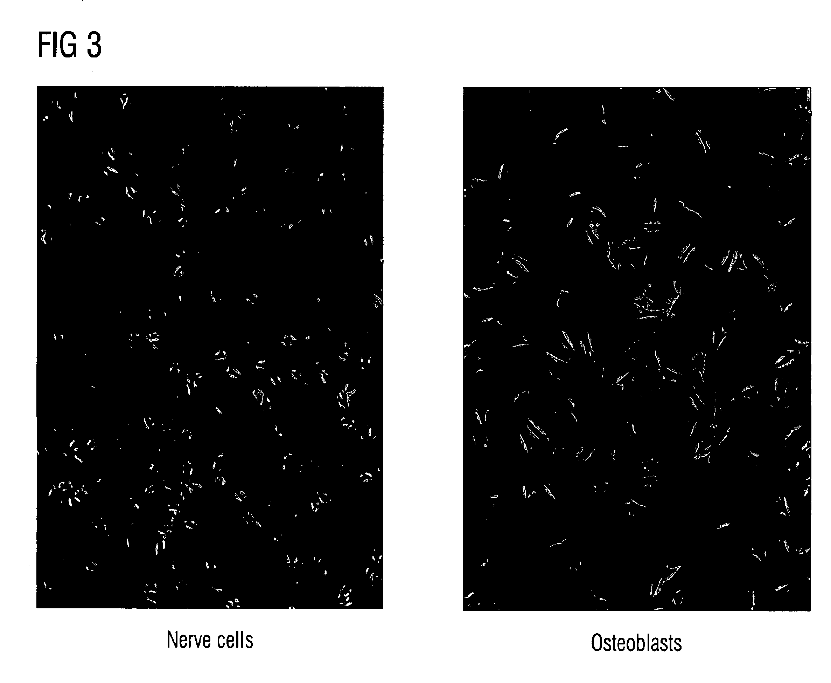

Furthermore, when osteoblast-derived nerve cells were transferred to a medium for osteoblasts, the nerve cells were differentiated into osteoblasts with a pavement stone shape (Fig. 3).

### Industrial Applicability

The present invention is a technology for transdifferentiating mature cells into other cells. The technology is useful for making it possible to use mature cells as a source for supplying cells in regenerative medicine, cell therapy, or the like. The use of the present invention eliminates the need of always using stem cells at medical sites; that is, situations where stem cells have been conventionally used. For example, the use of the present invention eliminates the need for performing procedures such as removal of stem cells from the bone marrow, umbilical cord blood, peripheral blood, placenta, or fat tissue, for example; selection of such cells; and use of such cells after storing them. Hence, necessary cells can be induced from finally differentiated mature cells that can be more conveniently collected than stem cells. Therefore, the present invention is a very useful technology at the sites of regenerative medicine, cellular medicine, and the like.

Furthermore, a transdifferentiation agent such as a demethylating agent is not used in the present invention. Hence, the method of the present invention is a method with a low risk of canceration and high safety. Moreover, random differentiation occurs infrequently in the case of the method of the present invention. Because of the resulting high stability and ease (the sole step required is to change media), the present invention has an advantage of being easily used clinically or easily applied industrially to automation, for example.

## Claims

1. A method for transdifferentiating a finally differentiated mature cell having a first differentiation phenotype into a finally differentiated mature cell having a second differentiation phenotype that differs from said first differentiation phenotype by changing culture conditions for the finally differentiated mature cell having the first differentiation phenotype, wherein culture conditions suitable for culturing the finally differentiated mature cell having said first differentiation phenotype are changed to culture conditions suitable for culturing the finally differentiated mature cell having said second differentiation phenotype.

2. The method according to claim 1 wherein the change of the culture conditions is a change of a medium.

3. The method according to claim 1 or 2 wherein transdifferentiation is performed without using a DNA methylating agent or a DNA demethylating agent.

4. The method according to any of claims 1 to 3 wherein the finally differentiated mature cell having the first differentiation phenotype is an osteoblast, and the finally differentiated mature cell having the second differentiation phenotype is a nerve cell.

5. The method according to claim 4 which comprises exchanging the medium from a medium containing β-glycerophosphate, ascorbic acid and Glutamax to a medium containing bFGF, FGF-8, EGF and BDNF.

6. A finally differentiated mature cell having a predetermined differentiation phenotype which is obtained by the method of any of claims 1 to 5.
